# EUROPEAN PATENT SPECIFICATION

(11) **EP 3 082 854 B1**
(45) Date of publication and mention of the grant of the patent: **17.10.2018**
(21) Application number: 14833390.9
(22) Date of filing: 18.12.2014
(51) Int. Cl.: A61K 39/00, A61K 39/39

(54) **METHOD FOR EMULSIFYING A TRIEPITOPE PEPTIDE WITH MONTANIDE AND KITS FOR PERFORMING THE SAME**
VERFAHREN ZUR EMULGIERUNG EINES TRIEPITOPPEPTIDS MIT MONTANID UND KITS ZU SEINER DURCHFÜHRUNG
PROCÉDÉ D'ÉMULSIFICATION D'UN PEPTIDE TRIEPITOPE AVEC MONTANIDE ET KITS PERMETTANT DE METTRE EN OEUVRE CELUI-CI

(30) Priority: 18.12.2013 EP 13306769
(43) Date of publication of application: 26.10.2016
(73) Proprietor: Vaxon Biotech, 75749 Paris Cedex 15 (FR)
(72) Inventor: MENEZ-JAMET, Jeanne, F-92120 Montrouge (FR)
(74) Representative: Santarelli
(86) International application number: PCT/IB2014/067095
(87) International publication number: WO 2015/092746

(56) References cited:
- WO-A1-2007/073768
- WO-A1-2010/112962
- "Vaccine Adjuvants. Your solution for therapeutic vaccines", Seppic , 1 February 2010 (2010-02-01), XP002724181, Retrieved from the Internet: URL:http://www.seppic.com/file/galleryelem ent/pj/80/0c/ca/2a/vaccine%20adjuvants%20l eaflet2413171879843931288.pdf;jsessionid=q kPO+z725ClGNJqfK+F2GQ__ [retrieved on 2014-05-09]
- KOTSAKIS A ET AL: "Clinical outcome of patients with various advanced cancer types vaccinated with an optimized cryptic human telomerase reverse transcriptase (TERT) peptide: Results of an expanded phase II study", ANNALS OF ONCOLOGY 2012 OXFORD UNIVERSITY PRESS GBR, vol. 23, no. 2, February 2012 (2012-02), pages 442-449, XP002724182, ISSN: 0923-7534
- STEPHANE ASCARATEIL ET AL: "New equipment optimized for emulsified vaccine prepation in the hospital", JOURNAL FOR IMMUNOTHERAPY OF CANCER, BIOMED CENTRAL LTD, LONDON, UK, vol. 1, no. Suppl 1, 7 November 2013 (2013-11-07), page P196, XP021167149, ISSN: 2051-1426, DOI: 10.1186/2051-1426-1-S1-P196 cited in the application
- "Technical Bulletin. Montanide ISA 50 V2 for antibody production", Seppic , 1 June 2008 (2008-06-01), XP002724183, Retrieved from the Internet: URL:http://www.seppic.com/file/galleryelem ent/pj/13/15/bf/55/leaflet%20montanide%20f or%20antibodies_production1673381072965306 38.pdf;jsessionid=+gu4zacrxQ078uCHoX2x3A__ [retrieved on 2014-05-09]
- ROSENBERG STEVEN A ET AL: "Different Adjuvanticity of Incomplete Freund's Adjuvant Derived From Beef or Vegetable Components in Melanoma Patients Immunized With a Peptide Vaccine", NIH Public Access Author Manuscript , July 2010 (2010-07), XP055097740, Retrieved from the Internet: URL:http://www.ncbi.nlm.nih.gov/pubmed/205 51834 [retrieved on 2014-05-12]

### SEQUENCE LISTING

<110> VAXON BIOTECH MENEZ-JAMET, Jeanne
<120> METHOD FOR EMULSIFYING A TRIEPITOPE PEPTIDE WITH MONTANIDE AND KITS FOR PERFORMING THE SAME
<130> VMA/sf-F1788/8-PCT
<150> EP13306769.4
   <151> 2013-12-18
<160> 1
<170> PatentIn version 3.5
<210> 1
   <211> 28
   <212> PRT
   <213> Artificial Sequence
<220>
   <223> polypeptide
<400> 1
   Tyr Leu Gln Val Asn Ser Leu Gln Thr Val Tyr Leu Glu Tyr Arg Gln
   1               5                   10                  15
   Val Pro Val Tyr Leu Glu Glu Ile Thr Gly Tyr Leu
               20                  25

## Description

The present invention pertains to the field of anti-cancer vaccines. More particularly, the invention relates to a method for preparing a vaccine formulation with an optimized polypeptide comprising three epitopes and Montanide as adjuvant, for use in anti-cancer immunotherapy.

WO2007073768 (A1) discloses a polypeptide having the sequence Vx006 (YLQVNSLQTVYLEYRQVPVYLEEITGYL, SEQ ID No: 1), which comprises three universal tumor-antigen-derived optimized cryptic peptides (TERT_{988Y}, MAGE-A_{248V9} and HER-2/neu_{402Y}). Contrary to a simple mixture of the three peptides and to other polypeptides comprising the same epitopes but in a different order, the polypeptide of SEQ ID No: 1 elicits a polyspecific CTL response against the native epitopes TERT₉₈₈, HER-2/neu₄₀₂, MAGE-A_{248G9} and MAGE-A_{248D9}, expressed by tumor cells. Since almost 100% of tumours express at least one of the three antigens, Vx006 thereby constitutes an excellent candidate for anticancer immunotherapy.

Immunological adjuvants are substances which are added to vaccine formulations to increase their efficacy. Adjuvants can increase the magnitude and duration of the immune response induced by vaccination. IFA (Incomplete Freund's adjuvant) is one of the most commonly used adjuvants in research. It is prepared from non-metabolizable oils. Vaccine formulations with IFA are water-in-oil (W/O) emulsions. IFA induces an immune response through the formation of a depot at the injection site and the stimulation of antibody producing plasma cells. However, adverse reactions, including sterile abscesses requiring surgical intervention in a number of cases, and some preclinical findings suggesting that IFA could be carcinogenic in laboratory animals, prevented further clinical development of IFA-adjuvanted vaccines. IFA-type detoxified adjuvants with improved specifications and quality control steps have then become available. For example, the mineral oil-based Montanide® ISA 51 and squalene-based Montanide® ISA 720 (SEPPIC, France) emulsions are currently undergoing clinical developments in vaccine studies against cancer, malaria and HIV. However, because W/O emulsions persist at the site of injection where they tend to cause local reactions, the application of these W/O emulsion adjuvants may remain limited to the field of therapeutic and cancer vaccines in humans. A vaccine against non-small-cell lung cancer, the CIMAVax® EGF vaccine using Montanide® ISA 51 VG, is undergoing Phase III studies and has already been licensed at least in Cuba.

Montanide® ISA 51 is a mixture of a highly purified mineral oil (Drakeol 6VR) and a surfactant (Mannide monooleate). In 2006, owing to concerns of prion contamination, the formulation of Montanide® ISA 51 was changed from using oleic acid isolated from beef tallow to that isolated from olives (Montanide® ISA 51 VG).

Process used to perform water in oil (W/O) emulsified vaccines can be obtained by a variety of protocols, using different devices such as high shear mixers, vortex, syringes, or syringes and connectors (T- or I-connectors). The methods used to obtain the emulsion are not all equivalent regarding vaccines physico-chemical parameters and the efficacy of vaccination.

Classically, to obtain an emulsified peptide with Montanide® ISA 51, two silicone-free syringes connected by a connector (I or T-connector) are used to bring a high shear in order to entrap droplets of water into surrounding oil phase. Typically, the peptide is dissolved in a saline solution (most often PBS or NaCl 0.9% saline buffer) and one volume of this solution is mixed with one volume of Montanide® ISA 51. The mix is then loaded into the device consisting of two silicone-free syringes connected for example by an I-connector, which is used to perform a pre-emulsification step of 20 low speed cycles (*i.e*., cycles lasting approximately 4 seconds), followed by a 40 rapid cycles emulsification step. A cycle is defined as the transfer of the whole solution (aqueous phase and adjuvant) from a first syringe to the other, followed by a transfer of the whole solution back to its syringe of origin. Recently, Ascarateil and Koziol presented the results obtained with a new equipment optimized for automatic W/O emulsion process (Journal for ImmunoTherapy of Cancer 2013). This device, disclosed in the patent application EP 2 322 132 A1, is an automated closed system allowing to control the emulsification process.

Kotsakis et al. (Annals of Oncology 2012, p.442-449) previously described a cryptic peptide vaccine Vx001 (YLFFYRKSV), obtained by dissolving 2 mg of peptide in 0.5 ml of sterile water and emulsifying this aqueous phase with Montanide ISA 51.

The inventors have tried to obtain W/O emulsions with Montanide® ISA 51 and an aqueous phase comprising the polypeptide of SEQ ID No: 1, by the classical protocol described above. When doing so, they encountered a number of unexplained difficulties which are illustrated in the experimental part below. Since the standard protocol for obtaining an emulsion with a peptide solution and Montanide® failed, the inventors tried a number of parameters variations in the emulsifying process, without success. Finally, they found that surprisingly, the polypeptide of SEQ No: 1 could be emulsified with Montanide® ISA 51 VG only if the two following conditions are fulfilled:
- the polypeptide of SEQ No: 1 must be dissolved in pure water instead of saline solution; and
- the pre-emulsification step must comprise at least 2 very slow cycles, each lasting at least 6 seconds.

The present invention hence pertains to a method for obtaining a water in oil emulsion for administration of a polypeptide of sequence YLQVNSLQTVYLEYRQVPVYLEEITGYL (SEQ ID No: 1) to a human subject, comprising the following steps:
(i) obtaining an aqueous antigenic phase by dissolving lyophilized polypeptide of SEQ No: 1 in pure water (water for injection);
(ii) loading one volume of said aqueous antigenic phase and one volume of Montanide into a device comprising two syringes linked by a connector;
(iii) pre-emulsifying the formulation by performing at least three cycles of transfer of the whole formulation from one syringe to the other and then back to the first syringe, wherein each cycle lasts at least 6 seconds; and
(iv) emulsifying the formulation by performing at least 15 additional transfer cycles, wherein each cycle lasts at most 2 seconds.

In the present text, "Montanide" designates the presently sold Montanide® ISA 51 VG, as well as any bioequivalent adjuvant derived therefrom (for example, by replacing the oleic acid isolated olives by that isolated from another source or a synthetic one).

It should also be kept in mind that in step (ii), the 50/50 ratio of water and oil phases can vary. Indeed, the adjuvant can represent up to 60 percents of the whole solution. Hence, unless otherwise specified, the phrase "mixing one volume of aqueous phase and one volume of Montanide" means "mixing aqueous phase and Montanide in a W/O ratio comprised between 50/50 and 40/60".

Importantly, the syringes used to perform the above process must be appropriate for Montanide, *i.e*., they must be silicon-free and rubber free (*i.e*., without any rubber tip free on the plunger), and preferably also latex-free. Examples of syringes which can be used to perform the invention are:
- OR 2 ml Injekt® (Ref: 4606701V from B-Braun, Germany),
- OR 5 ml Injekt® (Ref: 4606710V from B-Braun, Germany),
- OR 2 ml Norm-Ject (Ref: 4020.000V0 from Henke Sass Wolf GMBH, Germany),
- OR 5 ml Norm-Ject (Ref: 4050.000V0 from Henke Sass Wolf GMBH, Germany).

In the method according to the present invention, step (i) can be performed by hydrating a lyocake of polypeptide of SEQ No: 1 with pure sterile water and waiting until the dissolution is completed. When this step is performed at a temperature between 10 and 40°C, a few seconds to 2 minutes are necessary to obtain a complete dissolution of the lyocake; a gentle agitation of one to a few seconds, by taking the vial into two fingers, can be applied if necessary. The dissolution step can also be performed at a lower temperature, for example in the fridge (1-4°C).

According to a particular embodiment, step (i) is performed so that a volume of 0.5 to 6 ml, preferably around 1 ml or 5 ml of aqueous antigenic phase is obtained.

According to a particular embodiment, step (i) is performed so that the final concentration of peptide of SEQ ID No: 1 in the aqueous antigenic phase is between 2 and 20 mg/ml, preferably in the range of 2 to 10 mg /ml.

In a particular embodiment, step (ii) is performed by loading one volume of the aqueous antigenic phase into a first syringe and at least one volume of Montanide into a second syringe, and then bridging the two syringes by the connector, so that the final aqueous phase / Montanide ratio is comprised between 50/50 and 40/60. The simplest way to transfer liquids from vials is to use a syringe and a needle. However, this method has a risk of sting. Another safer solution is to use a vial adapter such as a 13 mm or a 20 mm vial adapters from West Pharmaceutical Services, Inc. or from Promepla (Monaco). For example, step (ii) can be performed by following the protocol described below:
- Remove the cover from the vial adapter package while handling the vial adapter through the blister package;
- Attach the adapter to the vial; use the blister package to handle the adapter. Seat the adapter on the vial by pushing it down until the spike penetrates the elastomeric stopper and the adapter snaps in place. Remove and discard the blister package;
- Twist the syringe onto the adapter;
- Withdraw 1 volume (typically, 1 ml) of sterile Montanide® ISA 51 VG into the syringe N°1 and remove air. Keep the syringe plugged on the vial;
- Repeat the same operation with a new vial adapter and the second syringe to withdraw 1 volume of the aqueous antigenic solution from the vial into syringe N° 2;
- Remove syringe N°2 from the adapter and connect it onto the connector;
- Push the plunger very slowly in order to drain the maximum of air from the system (this step can also be performed with syring N°1 instead of synringe N° 2);
- Remove syringe N°1 from the adapter and twist it onto the connector; so that the system is ready for emulsification.

According to a preferred embodiment, the connector used in step (ii) is an I-connector. Non-limitative examples of such connectors are:
- the I-connector developed by Green Peptide (Japan) for use with the emulsifying disclosed in the patent application EP 2 322 132 A1,
- the connector of reference DIDRACDLLFT from Didanorm (France),
- the I-connector (ref: ODG0015ST) from Promepla (Monaco), and
- the I-connector (ref: MX494) from Smiths medical (US), which is a non-DEHP formulation, latex-free, lipid resistant, non-PVC connector with and approximate priming volume of 0.2 ml and an approximate overall length of 5 cm.

As already mentioned, the pre-emulsification step (step (iii)) of the claimed method comprises at least two slow cycles, wherein each cycle corresponds to the transfer of the whole formulation (antigenic phase plus adjuvant), from one syringe to the other and then back to the first syringe and lasts at least 6 seconds; in a preferred embodiment, this step comprises 3 to 20 cycles, for example 3, 4, 5, 6, 7, 8, 9, 10 or 20 cycles. Each of these slow cycles preferably lasts between 6 and 30 seconds, more preferably between 8 and 15 seconds and even more preferably between 8 and 12 seconds, for example 8, 9, 10, 11 or 12 seconds. Remarkably, when the slow cycles are performed as indicated above, 5 slow cycles are sufficient for the pre-emulsion step, contrary to the typical protocol which comprises 20 slow cycles. Hence, at least when the pre-emulsification step is performed manually, the number of cycles of step (ii) is preferably between 3 and 8, for preferably between 4 and 6, and even more preferably 5.

The inventors have observed that once the pre-emulsification is correctly performed, a classical emulsification step produces a satisfying emulsion, even if no more than 15-20 fast transfer cycles are performed. Of course, an increased number of rapid cycles can be performed, such as at least 20 cycles, at least 30 cycles, at least 40 cycles and up to 60 cycles or even more. In what precedes, a "rapid" or "fast" cycle lasts between 0.5 and 2s at most 2 seconds, preferably less than 1.5s, more preferably less than 1s and even more preferably less than 0.7s (as fast as possible).

Also described herein is a kit for obtaining a water in oil emulsion containing the Vx006 polypeptide of SEQ ID No: 1 and Montanide, wherein said kit comprises at least 1 mg of lyophilized polypeptide of SEQ ID No: 1 (for example 2 mg or 10 mg), at least 1 ml of Montanide (for example, 3 ml of Montanide® ISA 51), two silicon- and rubber-free syringes, an I-connector and a notice of use describing the process according to any of the preceding claims. Optionally, the kit can also comprise a vial of water for injection and/or a vial adapter. A sterile needle can also be comprised, for vaccinating the patient after obtaining the emulsion. The syringes in the kit can be, for example, 2ml or 5 ml syringes. Optionally, the lyophilized Vx006 polypeptide can be replaced by a solution of Vx006 in sterile water.

Another aspect of the present invention is a water in oil emulsion containing the polypeptide of SEQ ID No: 1 and Montanide, wherein the peptide concentration in said emulsion is in the range 1 to 10 mg/ml.

Emulsions can be characterized by different parameters, including DvX, wherein X is comprised between 0 and 100, and which is the maximum particle size (diameter) for X% volume the sample. For example, Dv50 is the maximum particle diameter below which 50% of the sample volume exists, and Dv90 is the maximum particle diameter below which 90% of the sample volume exists. In a preferred embodiment of an emulsion according to the invention Dv 50 is below 3 µm, preferably below 2 µm and more preferably about 1.2 µm. For such emulsions, Dv90 is usually below 5 µm, preferably below 3 µm, and more preferably below 2 µm.

The invention is further illustrated by the following figure and examples.

### FIGURE LEGEND

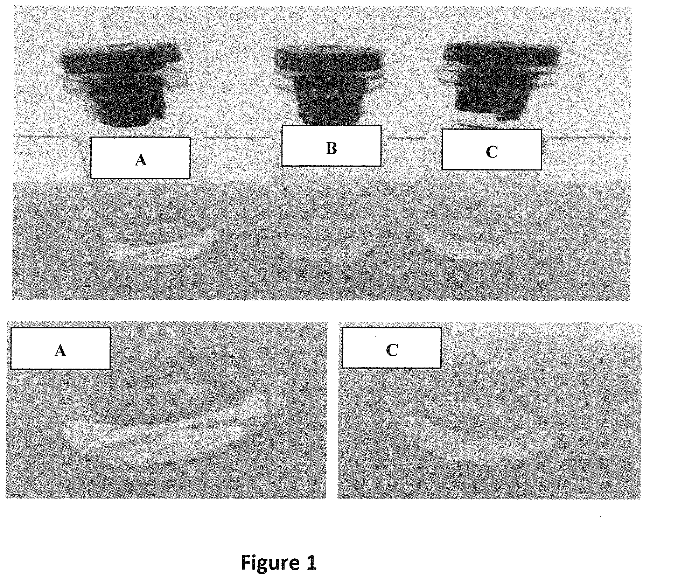

Figure 1: Vx006 in solution. A: in water for injection; B: in PBS buffer; C: in NaCl 0.9% saline buffer.

### EXAMPLES

The examples have been performed using the following materials and methods:
*Peptides.* Peptides were synthesized by BACHEM (Basel, Switzerland). Vx006 (SEQ ID No: 1) has been prepared in ammonium solution prior to lyophilisation.

*Montanide*® *ISA51VG.* Montanide® ISA51VG was provided by SEPPIC (Castres, France), Batch number T 134201.

*Micelle kun and emulsification devices.* A prototype of the Micelle kun (described in EP 2 322 132 A1), was used at SEPPIC (Castres, France). Silicone free syringes of 2ml are produced and marketed by BBraun (reference 4606701V). I- connector are produced and marketed by didactic GROUP (Etainhus, France) (reference RACDLLFTF).

*Emulsion characteristisation.* To characterize the emulsion, two tests are commonly used; the drop test and the measure of the drops size.

*Drop test*: The drop test allows checking the type of emulsion: W/O (aqueous phase drops in oily base) or O/W (drops of oil (adjuvant) in aqueous phase). In a beaker with clear water, lay 1 drop of emulsion on the surface of water and mix gently.

If the emulsion drop stays on the surface, it is a W/O emulsion.

If the emulsion disperses in water, it is an O/W emulsion.

*Size of drops:* The size of drops in the dispersed phase was checked by laser scattering granulometer. Particle size of emulsions was measured with a Malvern Mastersizer S. The average particle size was represented by:
- Dv 50 means:50% of the volumes of particles have a size lower than the Dv50,
- Dv 90 means: 90% of the volumes of particles have a size lower than the Dv90.

### Example 1: emulsification of placebo using the SEPPIC recommended protocol

Emulsifying Montanide® ISA 51 VG requires a saline aqueous phase. It is known that the use of saline buffer (such as PBS and NaCl 0,9%) allows producing thinner emulsions than pure water. To exemplify this, investors have compared emulsions prepared by five independent manipulators with Montanide® ISA 51 VG with either pure water or NaCl 0,9% saline solution. 0.7ml of placebo solution was loaded into a silicone free syringe and 0.7ml of Monatnide® ISA 51 VG into a second syringe. After connection of both syringes onto an I-connector, the classical protocol was applied, *i.e*., 20 slow cycles followed by 40 rapid cycles. The size of drops in the dispersed phase was checked by laser scattering granulometer. Particle size of emulsions was measured with a Malvern Mastersizer S.

Table 1 shows that Dv50 and Dv90 are significantly lower with NaCl 0,9% than with water. Dv50 and Dv90 measured with emulsion of NaCl 0,9% with Montanide® were 0.5 and 1.1 µm in average, compared to 0.8 and 1.7µm in emulsions with water. More importantly, emulsions performed with NaCl 0,9% saline buffer were much more stable than the emulsion with sterile water, since after 24 hours at 4°C, Dv50 and Dv90 measured with emulsions of NaCl 0,9% with Montanide® were respectively 1.1 and 1.5µm in average, compared to 3.6 and 8.3µm in emulsions with water. These results are in accordance with the recommendation of SEPPIC, Montanide® ISA51VG producer.

**Table 1: characteristics of emulsion performed with ISA51 VG with pure water or NaCl 0.9% saline buffer (ND: Not Done)**

| | Montanide ISA51 VG + NaCl 0,9% | | | | Montanide ISA51 VG + Pure water | | | |
|---|---|---|---|---|---|---|---|---|
| | T0 | | T24h | | T0 | | T24h | |
| | Dv50 | Dv90 | Dv50 | Dv90 | Dv50 | Dv90 | Dv50 | Dv90 |
| Manipulator 1 (test 1) | 0.7 | 1.4 | 0.8 | 1.4 | 1.1 | 1.6 | 3.6 | 8.2 |
| Manipulator 1 (test 2) | 0.3 | 0.6 | 0.3 | 0.7 | ND | | ND | |
| Manipulator 1 (test 3) | 0.4 | 1.3 | 0.5 | 1.1 | ND | | ND | |
| Manipulator 2 | 0.9 | 1.5 | 0.9 | 1.5 | 1.2 | 1.8 | 3.6 | 8.3 |
| Manipulator 3 | 0.5 | 1.1 | 0.5 | 1.1 | 1.3 | 1.8 | 3.7 | 8.4 |
| Manipulator 4 | 0.3 | 0.8 | 0.4 | 1.4 | ND | | ND | |
| Manipulator 5 | 0.3 | 0.8 | 2.3 | 4.4 | ND | | ND | |

### Example 2: Vx006 solubilisation

Vx006 was produced by BACHEM (Basel, Switzerland). For clinical trial, Vx006 was produced with a GMP grade, as vials containing 10mg of lyophilized peptide.

For vaccinating a patient, 1ml of solvent is added on the lyocake to resuspend the peptide, 0.7ml of peptide in solution is then loaded in a silicone free syringe and 0.7ml of Montanide® ISA51VG in a second syringe. Both syringes are then connected to the I-connector for the emulsification protocol.

In practice, solubilizing a peptide can be quite a challenge, although the following chemical rules exist to predict peptide solubility (disclosed, for example, in Sigma-Aldricht's web site):
1. Peptides which are shorter than 5 residues are generally soluble in aqueous media, except in extreme cases where all the residues are very hydrophobic (W, I, L, F, M, V or Y).
2. Hydrophilic peptides, containing >25% charged residues (E, D, K, R and H) and <25% hydrophobic residues also generally dissolve in aqueous media, provided that the charged residues are fairly distributed throughout the sequence. Peptides are generally purified with 0.1%TFA/water and 0.1% TFA/ACN solvent system. Therefore, if the peptide is dissolved in aqueous solution which is unbuffered or insufficiently buffered, the resulting peptide solution can be acidic. The pH of the solution must be around neutrality before trying other means of solubilization. Both acidic peptides (E+D residues> K+R+H residues) and basic peptides (R+K+H residues> E+D residues) are more soluble at neutral pH than at acidic pH.
3. Hydrophobic peptides containing 50% to 75% hydrophobic residues may be insoluble or only partially soluble in aqueous solutions, even if the sequence contains 25% charged residues. It is best to first dissolve these peptides in a minimal amount of stronger solvents such as DMF, acetonitrile, isopropyl alcohol, ethanol, acetic acid, 4-8M GdnHCl or urea, DMSO (if the sequence does not contain C, W or M), and other similar organic solvents, and then slowly add (drop wise) the solution to a stirred aqueous buffer solution. If the resulting peptide solution begins to show turbidity, the solubility limit might have been reached and it will be futile to proceed.
4. Very hydrophobic peptides containing >75% hydrophobic residues will generally not dissolve in aqueous solutions. These peptides generally require initial solubilization in very strong solvents such as TFA and formic acid and may precipitate when added into an aqueous buffered solution. The final peptide solution may require a higher concentration of organic solvent or denaturant, which may not be applicable in biological studies involving live cells.
5. Peptide sequences containing a very high (>75%) proportion of S, T, E, D, K, R, H, N, Q or Y are capable of forming extensive intermolecular hydrogen bonding network and have a tendency to form gels in concentrated aqueous solutions. These peptides may have to be treated similarly to step #3.

Since the Vx006 peptide of SEQ ID No: 1 comprises 15 hydrophobic residues (between 50 and 75%), 4 charged residues (< 25%) and 16 (< 75%) residues of the list cited in point 5 above, it theoretically needs a treatment as described in the above point 3, but may be partially soluble in water.

According to SEPPIC recommendations, to the fact that the emulsion is performed for human administration (preventing the use of solvents as DMSO *etc.*), results presented in Example 1 above and solubility prediction, Vx006 was initially diluted in saline buffer. Two saline solvents were tested, NaCl 0.9% and PBS. In both cases, the peptide in solution was cloudy, the solubilisation was not complete. More importantly and surprisingly, after a few minutes, the solution became viscous and finally a gel was formed, preventing any emulsification of the peptide with Montanide. Moreover, the addition of several solvents developed by Seppic and compatible with injection in humans and emulsion with Montanide were tested during the Vx006 manufacturing development. None of these solvents had any impact on the Vx006 solubilisation.

After a series of unsuccessful tries, inventors discovered that the peptide was perfectly soluble in water for injection, and that the pH of the obtained solubilised peptide was compatible with injection into humans (pH around 7). The lyocake was hence hydrated with a small volume of pure water (1 to 5 ml), and left at room temperature (15-25°C) until full dissolution (10 seconds to a few minutes). The vial can be gently agitated to accelerate the dissolution, if necessary. The dissolution can also be performed at cold temperature (0-10°C). The peptide in solution was totally clear and no gel formation was observed (Figure 1).

### Example 3: Vx006 emulsification in Montanide

Vx006 in solution in pure water at a concentration of 10mg/ml was then emulsified with Montanide® ISA51VG. First of all, 0.7ml of peptide in solution was loaded in a silicone free syringe and 0.7ml of Monatnide® ISA51VG in a second syringe. After connection of both syringes onto an I-connector, the typical protocol recommended by SEPPIC was applied, *i.e.,* 20 slow cycles (of 4 seconds) followed by 40 rapid cycles. Four manipulators trained for performing emulsion have applied the same protocol.

The drop test was then performed to evaluate the emulsions. As shown in table 2, in approximately 25% of the emulsifications, the emulsions were oil in water (O/W) emulsions, instead of water in oil (W/O). Moreover, even if the peptide was diluted 5 times (final concentration 2mg/ml), the result remained the same, showing that the emulsification capabilities of the peptide are not due to peptide concentration but to intrinsic characteristics of the Vx006 peptide. Such variability is not acceptable in the context of clinical trials, since oil in water emulsion do not play the role of adjuvant in the vaccination. Inventors then modified several parameters to define a robust protocol for emulsifying Vx006.

**Table 2: Emulsification of Vx006 in Montanide ISA 51 with the classical protocol.**

| Manupulator | Emulsion characteristics | | | |
|---|---|---|---|---|
| | Test 1 | Test 2 | Test 3 | Test 4 |
| M1 (10 mg/ml) | W/O | W/O | W/O | |
| M2 (10 mg/ml) | W/O | O/W | W/O | |
| M3 (10 mg/ml) | W/O | W/O | O/W | |
| M4 (10 mg/ml) | O/W | W/O | O/W | |
| M1 (2 mg/ml) | W/O | W/O | W/O | O/W |
| M3 (2 mg/ml) | W/O | W/O | O/W | |
| M4 (2 mg/ml) | O/W | O/W | W/O | W/O |

To avoid the manipulator bias, next tests were performed using a "Micelle kun", which is an automatized apparatus conceived to perform controlled emulsion processes (EP 2 322 132 A1). A prototype of the device was provided by SEPPIC. Three slow-cycle speeds were tested, "0" which corresponds to 12s for one cycle, "5" which corresponds to 6s for one cycle and "10", which is 4s per cycle. 20 slow cycles and 40 rapid cycles were then applied ("Micelle kun" performs the rapid cycles at the constant speed of 0.7s per cycle). Finally, both peptide concentrations 10mg/ml and 2mg/ml were tested. Results in table 3 show that "Micelle kun" was always able to produce W/O emulsions at speeds "0" and "5" but not at "10", showing the importance of applying very slow cycles during the pre-emulsifying step. It is important to note that the supplier of the "Micelle kun" initially envisaged to produce the "Micelle kun" with a unique "low" speed, but SEPPIC requested that the "low" speed can vary and reach cycles as long as 12 seconds, in order to ensure that it could be used to emulsify all peptides, including particular peptides such as Vx006. Indeed, the specialists from SEPPIC never worked with any peptide as sensitive to the parameters of the pre-emulsification step as the peptide of SEQ ID No:1.

**Table 3: Emulsification of Vx006 in Montanide® ISA 51 with various slow cycle speeds using the micelle kun.**

| Peptide concentration | Slow cycle speed | Drop test |
|---|---|---|
| 2 mg/ml | 0 | W/O |
| 2 mg/ml | 0 | W/O |
| 2 mg/ml | 0 | W/O |
| 2 mg/ml | 0 | W/O |
| 10mg/ml | 0 | W/O |
| 10 mg/ml | 0 | W/O |
| 10mg/ml | 5 | W/O |
| 10mg/ml | 5 | W/O |
| 10mg/ml | 5 | W/O |
| 10mg/ml | 5 | W/O |
| 10mg/ml | 5 | W/O |
| 2 mg/ml | 10 | O/W |

To simplify the preparation of the vaccine in the hospital, the inventors decided to determine the minimum number of slow cycles necessary to obtain a W/O emulsion. Using the "Micelle kun", the number of slow cycles was decreased. The protocol that allows to obtain each time W/O emulsions of Vx006 and Montanide® ISA 51 VG with the "Micelle kun" is: 5 cycles at very low speed (speed "0") followed by 40 cycles at high speed (table 4). It is to be noted that only 3 slow cycles can be sufficient, when these cycles are performed manually at a very low speed (at least 10-15 seconds per cycle).

**Table 4: determination of the minimal number of slow cycles**

| Peptide concentration | Slow cycle speed | Number of slow cycle + number of rapid cycle | Drop test |
|---|---|---|---|
| 2 mg/ml | 0 | 0+60 | O/W |
| 2 mg/ml | 0 | 3+40 | W/O |
| 2 mg/ml | 0 | 5+40 | W/O |
| 2 mg/ml | 0 | 10+40 | W/O |
| 2 mg/ml | 0 | 20+40 | W/O |
| 2 mg/ml | 10 | 3+40 | O/W |
| 2 mg/ml | 10 | 3+40 | W/O |
| 10 mg/ml | 0 | 3+40 | O/W |
| 10 mg/ml | 0 | 3+40 | W/O |
| 10 mg/ml | 0 | 5+40 | W/O |
| 10 mg/ml | 0 | 10+40 | W/O |
| 10 mg/ml | 0 | 20+40 | W/O |
| 10 mg/ml | 5 | 5+40 | W/O |

Finally, to test the robustness of the defined protocol, three manipulators applied 5 very slow cycles and 40 rapid cycles. Table 5 shows that 100% of the emulsions were W/O, thin and stable at 24 hours. Interestingly, the emulsions with Vx006 are more stable than those without the peptide (compare the data of Table 5 below with those of Table 1).

**Table 5: robustness of the protocol 5 + 40, performed manually**

| | Emulsion characteristic | Montanide ISA51 VG + Vx006 | | | |
|---|---|---|---|---|---|
| | | T0 | | T24h | |
| | | Dv50 | Dv90 | Dv50 | Dv90 |
| Manipulator 1 (10 mg/ml) Protocol 5+40 | W/O | 1.2 | 1.8 | 3.0 | 4.8 |
| Manipulator 2 (10 mg/ml) Protocol 5+40 | W/O | 1.2 | 1.7 | 2.9 | 4.5 |
| Manipulator 3 (10 mg/ml) Protocol 5+40 | W/O | 1.3 | 1.6 | 3.0 | 4.8 |

### REFERENCES

Ascarateil and Koziol: New equipment optimized for emulsified vaccine preparation in the hospital. Journal for ImmunoTherapy of Cancer 2013 1(Suppl 1):P196.

## Claims

1. A method for obtaining a water in oil emulsion comprising a polypeptide of sequence YLQVNSLQTVYLEYRQVPVYLEEITGYL (SEQ ID No: 1) and compatible with administration to a human subject, said method comprising the following steps:
(i) obtaining an aqueous antigenic phase by dissolving lyophilized polypeptide of SEQ No: 1 in pure sterile water;
(ii) loading a first volume of said aqueous antigenic phase and a second volume of Montanide into a device comprising two syringes linked by a connector, wherein the ratio of the first volume to the second volume is comprised between 50/50 and 40/60;
(iii) pre-emulsifying the formulation by performing at least three cycles of transfer of the whole formulation from one syringe to the other and then back to the first syringe, wherein each cycle lasts at least 6 seconds; and
(iv) emulsifying the formulation by performing at least 20 additional transfer cycles, wherein each cycle lasts at most 2 seconds.

2. The method according to claim 1, wherein step (i) is performed by hydrating a lyocake of polypeptide of SEQ ID No: 1 with pure sterile water at a temperature between 10 and 40°C and waiting until the dissolution is completed.

3. The method according to Claim 1 or claim 2, wherein the peptide concentration in the aqueous solution is between 2 and 20 mg/ml.

4. The method of any of the preceding claims, wherein step (ii) is performed by loading the first volume of aqueous antigenic phase into a first syringe and the second volume of Montanide into a second syringe, and then bridging the two syringes by the connector.

5. The method of any of the preceding claims, wherein the connector is an I connector.

6. The method of any of the preceding claims, wherein in step (iii), 3 to 10 cycles are performed.

7. The method of any of the preceding claims, wherein in step (iii), each cycle lasts between 6 and 30 seconds.

8. The method of any of the preceding claims, wherein in step (iv), between 20 and 40 cycles are performed.

9. The method of any of the preceding claims, wherein in step (iv), each cycle lasts between 0.5 and 2 seconds.

10. A water in oil emulsion containing the polypeptide of SEQ ID No: 1 and Montanide, wherein the peptide concentration in said emulsion is in the range 1 to 10 mg/ml, and wherein the Dv 50 of the emulsion is below 3 µm.

11. The water in oil emulsion of claim 10, wherein the Dv 50 of the emulsion is below 2 µm.

## Patentansprüche

1. Verfahren zum Erhalten einer Wasser-in-Öl-Emulsion, die ein Polypeptid der Sequenz YLQVNSLQTVYLEYRQVPVYLEEITGYL (SEQ ID No:1) umfasst und zur Verabreichung an ein menschliches Subjekt kompatibel ist, wobei das Verfahren die folgenden Schritte umfasst:
(i) Erhalten einer wässrigen antigenen Phase durch Lösen von lyophilisiertem Polypeptid der SEQ ID No:1 in reinem sterilem Wasser;
(ii) Laden eines ersten Volumens der wässrigen antigenen Phase und eines zweiten Volumens von Montanid in eine Vorrichtung, die zwei Spritzen umfasst, welche mittels eines Verbindungsstücks verbunden sind, wobei das Verhältnis des ersten Volumens zu dem zweiten Volumen zwischen 50/50 und 40/60 liegt;
(iii) Voremulgieren der Formulierung durch Durchführen von wenigstens drei Transferzyklen der gesamten Formulierung von einer Spritze zu der anderen und dann zurück zu der ersten Spritze, wobei jeder Zyklus wenigstens 6 Sekunden dauert, und
(iv) Emulgieren der Formulierung durch Durchführen von wenigstens 20 zusätzlichen Transferzyklen, wobei jeder Zyklus höchstens 2 Sekunden dauert.

2. Verfahren gemäß Anspruch 1, wobei Schritt (i) durch Hydratisieren eines Lyokuchens (lyocake) von Polypeptid mit SEQ ID No:1 mit reinem sterilem Wasser bei einer Temperatur zwischen 10 und 40°C und Warten, bis die Auflösung vollständig ist, durchgeführt wird.

3. Verfahren gemäß Anspruch 1 oder Anspruch 2, wobei die Peptidkonzentration in der wässrigen Lösung zwischen 2 und 20 mg/ml liegt.

4. Verfahren gemäß einem der vorangehenden Ansprüche, wobei Schritt (ii) durch Laden des ersten Volumens wässriger antigener Phase in eine erste Spritze und des zweiten Volumens von Montanid in eine zweite Spritze und danach Verbinden der zwei Spritzen mittels des Verbindungsstücks durchgeführt wird.

5. Verfahren gemäß einem der vorangehenden Ansprüche, wobei das Verbindungsstück ein I-Verbindungsstück ist.

6. Verfahren gemäß einem der vorangehenden Ansprüche, wobei in Schritt (iii) 3 bis 10 Zyklen durchgeführt werden.

7. Verfahren gemäß einem der vorangehenden Ansprüche, wobei in Schritt (iii) jeder Zyklus zwischen 6 und 30 Sekunden dauert.

8. Verfahren gemäß einem der vorangehenden Ansprüche, wobei in Schritt (iv) zwischen 20 und 40 Zyklen durchgeführt werden.

9. Verfahren gemäß einem der vorangehenden Ansprüche, wobei in Schritt (iv) jeder Zyklus zwischen 0,5 und 2 Sekunden dauert.

10. Wasser-in-Öl-Emulsion, die das Polypeptid mit SEQ ID No:1 und Montanid enthält, wobei die Peptidkonzentration in der Emulsion im Bereich von 1 bis 10 mg/ml liegt und wobei Dv 50 der Emulsion unter 3 µm ist.

11. Wasser-in-Öl-Emulsion gemäß Anspruch 10, wobei Dv 50 der Emulsion unter 2 µm ist.

## Revendications

1. Méthode pour obtenir une émulsion eau dans l'huile comprenant un polypeptide de séquence YLQVNSLQTVYLEYRQVPVYLEEITGYL (SEQ ID N°1) et compatible avec l'administration à un sujet humain, ladite méthode comprenant les étapes suivantes ;
(i) obtenir une phase antigénique aqueuse par la dissolution du polypeptide de SEQ ID N°1 lyophilisé dans de l'eau stérile pour injection ;
(ii) remplir un premier volume de ladite phase antigénique aqueuse et un second volume de Montanide dans un dispositif comprenant deux seringues reliées par un connecteur, où le ratio du premier volume sur le second volume est compris entre 50/50 et 40/60;
(iii) pré-émulsifier la formulation en réalisant au minimum trois cycles de transfert de toute la formulation d'une seringue à l'autre puis à nouveau dans la première seringue, chaque cycle durant au minimum 6 secondes ; et
(iv) émulsifier la formulation en réalisant au minimum 20 cycles de transfert supplémentaires, chaque cycle durant moins de deux secondes.

2. Méthode selon la revendication 1, dans laquelle l'étape (i) est réalisée en hydratant un lyophilisat de polypeptide de SEQ ID N°1 avec de l'eau stérile pour injection à une température comprise entre 10 et 40°C et en attendant jusqu'à la dissolution complète.

3. Méthode selon la revendication 1 ou la revendication 2, dans laquelle la concentration du peptide dans la phase antigénique aqueuse est comprise en 2 et 20mg/ml.

4. Méthode selon l'une quelconque des revendications précédentes, dans laquelle l'étape (ii) est réalisée en chargeant le premier volume de la phase antigénique aqueuse dans une première seringue et le second volume de Montanide dans une deuxième seringue puis en reliant les deux seringues avec le connecteur.

5. Méthode selon l'une quelconque des revendications précédentes, dans laquelle le connecteur est un connecteur en I.

6. Méthode selon l'une quelconque des revendications précédentes, dans laquelle durant l'étape (iii), 3 à 10 cycles sont réalisés.

7. Méthode selon l'une quelconque des revendications précédentes, dans laquelle durant l'étape (iii), chaque cycle dure entre 6 et 30 secondes.

8. Méthode selon l'une quelconque des revendications précédentes, dans laquelle durant l'étape (iv), 20 à 40 cycles sont réalisés.

9. Méthode selon l'une quelconque des revendications précédentes, dans laquelle durant l'étape (iv), chaque cycle dure entre 0,5 et 2 secondes.

10. Emulsion eau dans l'huile contenant le polypeptide de SEQ ID N°1 et du Montanide, dans laquelle la concentration du peptide dans ladite émulsion est comprise entre 1 et 10 mg/ml et dans laquelle la Dv50 de l'émulsion est inférieure à 3µm.

11. Emulsion eau dans l'huile selon la revendication 10, dans laquelle la Dv50 de l'émulsion est inférieure à 2µm.
